# EUROPEAN PATENT SPECIFICATION

(11) **EP 0 527 050 B1**
(45) Date of publication and mention of the grant of the patent: **16.10.1996**
(21) Application number: 92307167.4
(22) Date of filing: 05.08.1992
(51) Int. Cl.: A61N 5/06

(54) **Cancer therapy system**
Krebstherapiesystem
Système pour la thérapie du cancer

(30) Priority: 05.08.1991 JP 219189/91
(43) Date of publication of application: 10.02.1993
(73) Proprietor: ISHIKAWAJIMA-HARIMA JUKOGYO KABUSHIKI KAISHA, Chiyoda-ku Tokyo-to (JP); Kato, Harubumi, Shinjuku-ku, Tokyo-to (JP)
(72) Inventor: Udagawa, Takeshi, Kawasaki-shi, Kanagawa-ken (JP); Amano, Takeyasu, Yokohama-shi, Kanagawa-ken (JP); Shiozaki, Hiroyuki, Yokosuka-shi, Kanagawa-ken (JP); Mori, Mikio, Musashino-shi, Tokyo-to (JP); Degawa, Sadao, Komae-shi, Tokyo-to (JP); Kato, Harubumi, Shinjuku-ku, Tokyo-to (JP)
(74) Representative: Jennings, Nigel Robin

(56) References cited:
- EP-A- 0 429 297
- GB-A- 2 126 717
- PATENT ABSTRACTS OF JAPAN vol. 15, no. 184 (P-1200)13 May 1991 & JP-A-03 042 550 ( TOSOH ) 22 February 1991
- LEOS '90. IEEE LASERS AND ELECTRO-OPTICS SOCIETY ANNUAL MEETING. CONFERENCE PROCEEDINGS. vol. 2, 4 November 1990, NEW YORK, US pages 524 - 525 H.H. ZENZIE ET AL. 'Harmonic generation of Ti:Sapphire Lasers'
- H.W.Messenger et al.:"World laser review: foucs is on diode and solid state lasers" in Laser Focus World, Dec.1990, pp.55-76

## Description

The present invention relates to a cancer therapy system.

Japanese Patent 2nd Publication No. 63-2633 discloses a cancer therapy system using a pulsed laser beam in which the laser beam with a predetermined wavelength is radiated onto a cancerous focus onto which a photosensitive substance having an affinity with cancer tissue has been absorbed, thereby destroying the focus.

Japanese Patent 2nd Publication No. 63-9464 discloses a cancer diagnosis system using a pulsed laser beam in which the laser beam with a predetermined wavelength is radiated onto a focus onto which a photosensitive substance having an affinity with cancer tissue has been absorbed and fluorescence from the photosensitive substance is subjected to a fluorescence analysis to thereby diagnose whether a cancer is present on the target area.

In these known systems, an excimer laser is used to pump a dye laser so as to produce a laser beam with a predetermined wavelength.

The laser medium used in dye lasers, which is a liquid such as alcohol or a solvent having an organic pigment dye dissolved therein, has a short service life so that the pigment dye has to be frequently replaced. Moreover, the mirrors in excimer lasers have to be frequently cleaned.

EP-A-0429297 relates to a cancer diagnosis and treatment device in which a solid state laser generator such as an Nd:YAG laser or a titanium-sapphire laser emits a first laser beam which passes through a harmonic generator generating second and third harmonics the higher harmonic being passed through an optical parametric oscillator and being converted into second and third laser beams having respective wavelengths suitable for stimulating a sterilising photo-chemical reaction in a photosensitive material in a cancerous focus and inducing fluorescence in the photosensitive material.

In an article entitled "World Laser Review: Focus is on diode and solid-state lasers" from an issue of Laser Focus World dated December 1990, the possibility of using Nd:YAG lasers to pump titanium-sapphire lasers is discussed.

GB 2126717 describes a device for diagnosing cancers using a pulse laser beam source to cause fluorescence of a photosensitive material absorbed by a cancerous focus. Fluorescence from the cancerous focus is observed by an image detecting means via an endoscope and displayed on an imaging device.

It is the object of the present invention to overcome the above and other problems encountered in the prior art and to provide a cancer therapy system which utilizes a solid-state laser and is easy to operate and maintain.

According to the invention there is provided a cancer diagnosis and therapy system for a cancerous focus that has absorbed a photosensitive substance comprising:
a solid state laser unit including a YAG laser oscillator for producing a YAG laser fundamental wave;
a YAG laser second harmonic generator for producing a YAG laser second harmonic from the fundamental wave produced by the oscillator;
a YAG laser third harmonic generator for generating the YAG laser third harmonic;
an optical parametric oscillator for generating an optical parametric oscillator laser beam from the YAG laser third harmonic generated by the generator;
the optical parametric oscillator laser beam being of such a wavelength to destroy the photosensitive substance laden cancerous focus; and
a fibrescope for irradiating cancerous focuses with the laser beam generated by the laser unit and observing the focuses and an image display unit for viewing the focuses through the fibrescope and displaying an image of the focuses characterised in that:
the YAG laser third harmonic generator generates the YAG laser third harmonic from that proportion of the YAG laser fundamental wave which is not converted to the second harmonic by the generator and from the YAG second harmonic generated by the generator, and the solid state laser unit further comprises a titanium-sapphire laser oscillator which is pumped by the YAG laser second harmonic generator to generate a titanium-sapphire laser fundamental wave, a titanium-sapphire laser second harmonic generator for generating a titanium-sapphire laser second harmonic from the titanium-sapphire laser fundamental wave generated by the oscillator, the titanium-sapphire laser second harmonic being of such a wavelength to cause fluorescence of the photosensitive substance.

The image display unit for viewing the focuses through the fibrescope may serve to graphically display the spectrum of fluorescence from the focuses or fluorescent positions on said focuses.

The laser beam to be conducted to the focuses is produced by the solid-state laser unit so that the operating life is increased and maintenance is substantially facilitated.

Further features and details of the invention will be apparent from the following description of certain specific embodiments which is given by way of example with reference to the accompanying schematic drawings, in which:-
Figure 1 is a diagram of a first embodiment of the present invention;
Figure 2 is a diagram of the solid-state laser unit shown in Figure 1;
Figure 3 is a diagram of a second embodiment of the present invention;
Figure 4 is a diagram of a third embodiment of the present invention;
Figure 5 is a diagram of the solid-state laser unit shown in Figure 4; and
Figure 6 is a schematic diagram of a fourth embodiment of the present invention.

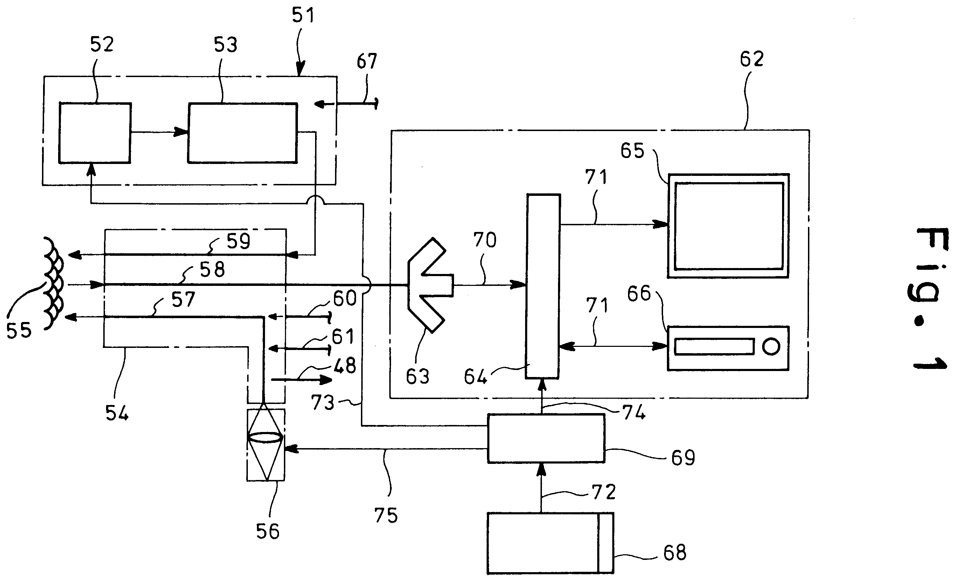

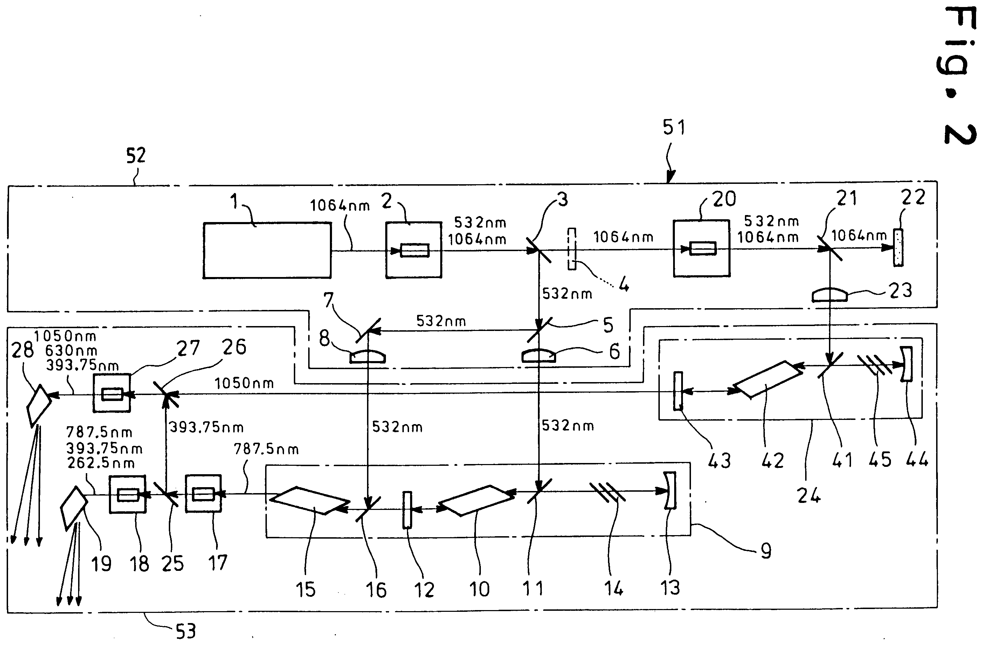

Figures 1 and 2 show a first embodiment of a cancer therapy system in accordance with Claim 1.

A solid-state laser unit, generally indicated by numeral 51, comprises a YAG laser 52 which emits a laser beam with a predetermined wavelength and a titanium-sapphire laser 53 which is pumped by the laser beam from the YAG laser 52 to emit a laser beam with a predetermined wavelength.

The laser unit 51 is connected to a supply pipe 67 through which cooling water is supplied to the laser 52 so as to prevent overheating of the latter.

A fibrescope, generally designated by numeral 54, is used for observation and treatment of cancerous focuses 55 and comprises an illuminating optical fibre 57 which is optically connected to a high-intensity illuminating source, such as a xenon arc lamp 56, to illuminate a focus 55, a picturing or imaging optical fibre 58 through which an operator, physician or the like observes the focus 55 and an irradiating optical fibre 59 through which the laser beam from the laser unit 51 is transmitted to the focus 55.

The fibrescope 54 is provided with supply pipes 60 and 61 through which cleaning water and air are respectively supplied to the tips of the fibres 57, 58 and 59 to clean them and a discharge pipe 48 for discharging the waste water and air.

An image display unit, generally indicated by numeral 62, is used to observe or diagnose the focus 55 and comprises a colour video camera 63 for viewing the focus 55 through the fibre 58 and delivering a video signal 70, a video image processor 64 for converting the signal 70 from the camera 63 into a processed video signal 71, a monitor screen 65 which receives the signal 71 from the processor 64 and displays the image of the focus 55 and a video recording/reproducing device 66, such as a video tape recorder, which receives and records the signal 71 from the processor 64 and transmits the recorded signal 71 to the processor 64 so that the recorded image of the focus 55 may be displayed on the screen 65, when so required.

The first embodiment further includes a control panel 68 which is manually operated by an operator to issue control signals 72 to control the solid-state laser unit 51, the image display unit 62 and the xenon arc lamp 56. In response to the signals 72 from the panel 68, a controller 69 delivers control signals 73, 74 and 75 to the laser 52, the processor 64 and the lamp 56, respectively, thereby activating or deactivating the units 51, 62 and the lamp 56, respectively.

Referring next to Figure 2, the YAG laser 52 in the solid-state laser unit 51 includes a YAG laser oscillator 1 which generates a laser beam or YAG laser fundamental wave with a wavelength of 1064 nm to be used for pumping. In response to the laser fundamental wave from the oscillator 1 a YAG laser second harmonic generator 2 generates a laser beam with a wavelength of 532 nm or YAG laser second harmonic in accordance with the following equation:$\frac{\text{1}}{{\text{λ}}_{\text{2}}} \text{=} \frac{\text{1}}{{\text{λ}}_{\text{1}}} \text{+} \frac{\text{1}}{{\text{λ}}_{\text{1}}}$ where λ₁ is the wavelength of the fundamental wave and λ₂ is the wavelength of the second harmonic. A dichroic mirror 3, on which that proportion of the YAG laser fundamental wave from the oscillator 1 which has not been converted into the second harmonic and the YAG laser second harmonic from the generator 2 is incident, transmits only the fundamental wave and reflects the second harmonic. A semi-reflective mirror 5 transmits part of the second harmonic reflected from the mirror 3 and reflects the remainder of the second harmonic. A lens 6 converges or focuses the second harmonic which has transmitted through the semi-reflective mirror 5. A total reflection mirror 7 reflects the second harmonic reflected from the semi-reflective mirror 5 onto a lens 8 which converges or focuses it.

The YAG laser fundamental beam transmitted by the mirror 3 is incident on a second harmonic generator 20 which, similarly to the generator 2, generates a laser beam with a wavelength of 532 nm (or YAG laser second harmonic) in accordance with relation (1) above. The beam leaving the second harmonic generator 20 is incident on a dichroic mirror 21 which transmits that proportion of the YAG laser fundamental wave which has not been converted into the second harmonic by the generator 20 and reflects the second harmonic generated by the generator 20. The transmitted beam is incident on a beam damper 22 which cuts off or attenuates the YAG laser fundamental wave. The reflected beam is incident on a lens 23 which converges or focuses it.

It is to be noted that a beam damper 4 capable of cutting off or attenuating the fundamental wave with a wavelength of 1064 nm may be interposed between the mirror 3 and the generator 20.

Referring still to Figure 2, the titanium-sapphire laser 53 in the solid-state laser unit 51 comprises a titanium-sapphire laser oscillator 9 which includes a dichroic mirror 11 which reflects the YAG laser second harmonic converged by the lens 6 and transmits beams with other wavelengths. The reflected beam is incident on a titanium-sapphire laser rod 10 which is pumped in response to the incident of the reflected YAG laser second harmonic to generate a beam with a wavelength of 670 to 1100 nm. Front and rear mirrors 12 and 13 define a resonator in which the beam from the rod 10 resonates. Situated within the resonator is a wavelength tuning or selection element 14, such as a double refraction filter, which transmits light with a predetermined wavelength and absorbs light with other wavelengths. A dichroic mirror 16 transmits the titanium-sapphire laser fundamental wave with a wavelength between 670 and 1100 nm after it has reached a predetermined intensity during the resonance between the mirrors 12 and 13 and reflects the YAG laser second harmonic converged by the lens 8. A titanium-sapphire laser amplification rod 15 is pumped in response to the incidence of the YAG laser second harmonic reflected from the mirror 16 to amplify the intensity of the incident titanium-sapphire fundamental wave which passes through the mirror 16.

With a laser oscillator 9 of the type described above, the wavelength of the emitted titanium-sapphire laser fundamental wave can be arbitrarily selected within the wavelength range 670 to 1100 nm by angularly displacing the element 14.

The beam emitted by the amplification rod 15 is incident on a second harmonic generator 17 which generates the titanium-sapphire laser second harmonic from the titanium-sapphire fundamental wave emitted from the rod 15 in accordance with relation (1) referred to above. A titanium-sapphire laser third harmonic and fourth harmonic generator 18 which generates the titanium-sapphire laser third harmonic from the titanium-sapphire laser second harmonic generated by the generator 17 and the titanium-sapphire laser fundamental wave which was not converted into the second harmonic in accordance with the following relation$\frac{\text{1}}{{\text{λ}}_{\text{3}}} \text{=} \frac{\text{1}}{{\text{λ}}_{\text{1}}} \text{+} \frac{\text{1}}{{\text{λ}}_{\text{2}}}$ where
- λ₁:: wavelength of fundamental wave,
- λ₂:: wavelength of second harmonic and
- λ₃:: wavelength of third harmonic
or generates the titanium-sapphire laser fourth harmonic (second harmonic of second harmonic) from the titanium-sapphire laser second harmonic in accordance with relation (1) referred to above.

The generator 18 includes a crystal cell containing a non-linear optical crystal capable of generating the third harmonic or a crystal cell containing a non-linear optical crystal capable of generating the fourth harmonic. It follows therefore that exchanging the crystal cells enables generation of the titanium-sapphire laser third or fourth harmonic.

Reference numeral 19 denotes a pellinbroca prism for separating the titanium-sapphire laser fundamental wave and second harmonic which have not been converted by the generator 18 and the titanium-sapphire laser third or fourth harmonic generated by the generator 18.

A difference-frequency-generating titanium-sapphire laser oscillator, generally designated by numeral 24, generates a difference frequency and comprises a dichroic mirror 41 which reflects the YAG laser second harmonic converged by the lens 23 and transmits beams with other wavelengths. The second harmonic reflected by the mirror 42 is incident on a titanium-sapphire laser rod 42 which is pumped by it to generate a beam with a wavelength between 670 and 1100 nm. Front and rear mirrors 43 and 44 define a resonant chamber for the beam emitted from the rod 42. A wavelength tuning or selection element 45, such as a double refraction filter, which only passes beams with a predetermined wavelength and scatters beams with other wavelengths is provided in the resonant pathway.

The wavelength of the fundamental wave emitted from the oscillator 24 can be arbitrarily selected within the wavelength range of 670 to 1100 nm by angularly displacing the element 45.

A dichroic mirror 25 is removably interposed between the generators 17 and 18 and transmits only the titanium-sapphire laser fundamental wave which has not been converted to the second harmonic by the generator 17 and reflects the titanium-sapphire laser second harmonic generated by the generator 17. A dichroic mirror 26 reflects the second harmonic from the mirror 25 and transmits only the titanium sapphire laser fundamental wave from the oscillator 24. A difference frequency generator 27 produces a difference frequency laser beam from the titanium-sapphire laser second harmonic reflected from the mirror 26 and the titanium-sapphire laser fundamental wave transmitted through the mirror 26 in accordance with the following relation$\frac{\text{1}}{{\text{λ}}_{\text{6}}} \text{=} \frac{\text{1}}{{\text{λ}}_{\text{5}}} \text{+} \frac{\text{1}}{{\text{λ}}_{\text{4}}}$ where
- λ₆:: wavelength of differential frequency wave,
- λ₄:: wavelength of beam wave A,
- λ₅:: wavelength of beam wave B and λ4 > λ5.
A pellinbroca prism 28 separates the titanium-sapphire laser second harmonic (beam B) remaining downstream of the generator 27, the titanium-sapphire laser fundamental wave (beam A) also remaining downstream of the generator 27 and the difference frequency laser beam emitted from the generator 27.

The mode of operation of the first embodiment is as follows:-

When a cancerous focus 55 is to be subjected to therapy, the optical characteristics of the wavelength tuning element 14 in the oscillator 9 are so adjusted that a laser beam with a wavelength of 787.5 nm is permitted to pass and beams with other wavelengths are absorbed. The optical characteristics of the wavelength tuning element 45 in the difference-frequency-generating titanium-sapphire laser 24 are also so adjusted that a laser beam with a wavelength of 1050 nm is permitted to pass through and laser beams with other wavelengths are absorbed. Then, the dichroic mirror 25 is interposed between the generators 17 and 18.

A hematoporphyrin derivative (hereinafter referred to as HPD), which has affinity with cancerous tissue and is photosensitive, is injected into a blood vessel of the patient and the HPD is preferentially absorbed by the focus 55 and is scarcely absorbed by normal tissue.

When irradiated with a laser beam with a wavelength of 630 nm, HPD-laden cancerous focuses 55 are destroyed, whereas normal tissue with virtually no HPD absorbed therein is scarcely affected at all by irradiation with the same laser beam.

After the focus 55 has absorbed HPD, the fibrescope 54 is positioned such that its tip is substantially opposed to the focus 55.

The control panel 68 is now manually operated to transmit the command signal 72 to the controller 69 to activate the image display unit 62 and the arc lamp 56, so that the controller 69 transmits the command signals 74 and 75 to the video image processor 64 and the lamp 56, respectively, thereby activating them. The beam from the lamp 56 then illuminates the focus 55. The image of the focus 55 is picked up by the colour video camera 63 and displayed on the monitor screen 65 thereby enabling the focus 55 to be observed and diagnosed.

The control panel 68 is then manually operated again to transmit the command signal 72 to the controller 69 which transmits the command signal 73 to the YAG laser 52 in the solid-state laser unit 51 to energise it. In response to the command signal 73, the YAG laser oscillator 1 is energised so that a laser beam with a wavelength of 1064 nm (the YAG laser fundamental wave) is produced. In response to the laser beam thus produced, the generator 2 generates the YAG laser second harmonic with a wavelength of 532 nm which is then reflected by the mirror 3. Part of the YAG laser second harmonic reflected from the mirror 3 passes through the semi-reflective mirror 5, the lens 6 and the mirror 11 and enters the laser rod 10 of the oscillator 9 so that the titanium-sapphire laser fundamental wave with a wavelength between 670 and 1100 nm is produced.

The fundamental wave passes through the rod 10 again due to the presence of the mirrors 12 and 13. Thereafter, only the fundamental wave with a wavelength of 787.5 nm passes through the wavelength tuning element 14 while fundamental waves with other wavelengths are absorbed by the element 14. When the intensity of the titanium-sapphire laser fundamental wave with a wavelength of 787.5 nm reaches a predetermined level, the titanium-sapphire fundamental wave passes through the mirrors 12 and 16 and enters the laser amplification rod 15.

Meanwhile, part of the YAG laser second harmonic reflected by the mirror 3 is reflected by the semi-reflective mirror 5 toward the total reflection mirror 7 which in turn reflects it to the lens 8. Having passed through the lens 8, the YAG laser second harmonic enters the laser amplification rod 15 in the oscillator 9 as the pumping beam so that the rod 15 is pumped and the titanium-sapphire laser fundamental wave with a wavelength of 787.5 nm emitted from the laser rod enters the amplification rod 15 and is amplified.

The titanium-sapphire laser fundamental wave with a wavelength of 787.5 nm and amplified by the rod 15 enters the titanium-sapphire laser second harmonic generator 17 which generates the titanium-sapphire laser second harmonic with a wavelength of 393.75 nm in accordance with relation (1) above. The titanium-sapphire laser second harmonic generated by the generator 17 upon incidence of the titanium-sapphire laser fundamental wave is reflected by the mirror 25 whereas the titanium-sapphire laser fundamental wave which passes through the generator 17 is transmitted through the dichroic mirror 25.

The second harmonic reflected by the mirror 25 is reflected by the mirror 26 into the difference frequency generator 27.

Meanwhile, the YAG laser fundamental wave which is transmitted through the mirror 3 enters the difference-frequency -generating YAG laser second harmonic generator 20 which generates the YAG laser second harmonic with a wavelength of 532 nm in accordance with relation (1) above. The YAG laser second harmonic thus generated is directed as the pumping beam by the optical elements including the dichroic mirror 21 and the lens 23 to the difference-frequency-generating titanium-sapphire laser oscillator 24 which generates the titanium-sapphire laser fundamental wave with a wavelength of 1050 nm which in turn is transmitted through the dichroic mirror 26 to the difference frequency generator 27.

In response to the incidence of the titanium-sapphire laser fundamental wave with a wavelength of 1050 nm and the titanium-sapphire laser second harmonic with a wavelength of 393.75 nm, the difference frequency generator 27 generates a laser beam with a wavelength of 630 nm in accordance with relation (3) above. That proportion of the titanium-sapphire laser fundamental wave and of the second harmonic transmitted through the generator 27 and the laser beam with the wavelength of 630 nm generated by the generator 27 are separated from each other by the prism 28.

The laser beam with the wavelength of 630 nm separated by the prism 28 is transmitted through the irradiating optical fibre 59 in the fibrescope 54 and is radiated onto the cancerous focus 55, which, due to the fact that HPD is absorbed in the cancerous tissue, is destroyed by the laser beam.

The first embodiment has been described with reference to the use of HPD. If other photo-sensitive substances are used, the optical characteristics of the wavelength tuning elements 14 and 45 are varied to match the wavelengths of the laser beams generated by the titanium-sapphire laser second harmonic generator 17 and the difference frequency generator 27 with the characteristic or absorption band of the photosensitive substance actually used.

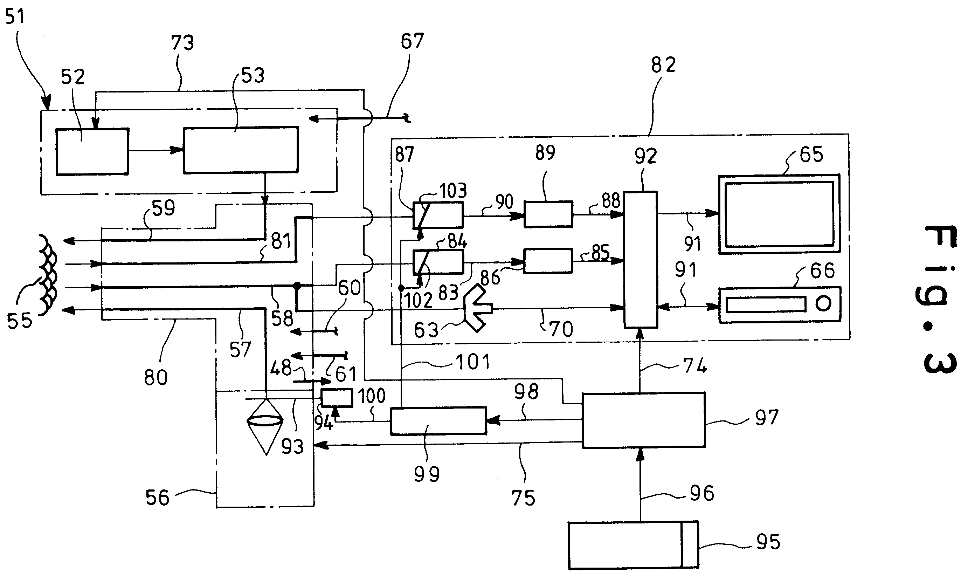

Figure 3 is a schematic diagram of a second embodiment of the present invention in accordance with Claims 2 and 3. The solid-state laser unit 51 shown in Figure 3 is substantially similar to that described above and those parts which are similar to those shown in Figures 1 and 2 are designated by the same reference numerals.

Reference numeral 80 denotes a fibrescope for observing, diagnosing and treating cancerous focuses 55 which is substantially similar to the fibrescope 54 shown in Figure 1 except that it further incorporates a diagnosing optical fibre 81.

The xenon arc lamp 56 which generates light to be transmitted through the fibrescope 80 incorporates an optical chopper 93 so that the beam for illuminating the focuses 55 can be transmitted intermittently.

The optical chopper 93 has a disk which is formed with a plurality of holes arranged in a desired pattern and is rotated by a motor 94 so that light from the lamp 56 is transmitted intermittently to the focuses 55.

An image display unit, generally designated by numeral 82, for observing and diagnosing the focuses 55 comprises a colour video camera 63 which picks up the image of a focus 55 through an imaging fibre 58 and produces a video output signal 70. An optical amplifier 84 is arranged to receive fluorescence from the focus 55 through the fibre 58 and amplifies the received fluorescence and produces an amplified fluorescence signal 83. A high-sensitivity camera 86 converts the signal 83 from the amplifier 84 into a video signal 85. A fluorescence spectroscope 87 receives the fluorescence from the focus 55 through the diagnosing optical fibre 81 and resolves fluorescent radiation with a predetermined wavelength. An optical amplifier 89 receives the fluorescent radiation 90 from the spectroscope 87, amplifies it and produces a video signal 88. A video image processor 92 receives the signals 70, 85 and 88 from the cameras 63 and 86 and amplifier 89, respectively, and converts them into a processed video signal 91 which is supplied to a monitor screen 65, which displays the image of the focus 55, the image of fluorescent positions on the focus 55 or the fluorescent spectrum from the focuses 55a, and to a recording/reproducing unit 66 for recording the processed video signal 91 and transmitting it to the monitor screen 65 through the processor 92, to display the image of the focus 55, the image of the fluorescent positions on the focus 55 or the fluorescent spectrum from the focus 55.

As shown in Figure 3, the second embodiment further includes a control panel 95 which is manually operated to transmit a command signal 96 for activating the solid-state laser unit 51, the video image display unit 82, the xenon arc lamp 56 and the optical chopper 93, depending upon whether observation, diagnosis and/or treatment is to be carried out. The command signal 96 is received by a computer 97 which then delivers an actuating signal 73 to the YAG laser 52 to energise the solid-state laser unit 51 and an actuating signal 74 to the processor 92 to energise the image display unit 82 and an actuating signal 75 to the lamp 56 to energize it.

The computer 97 also produces a switching signal 98 depending upon whether it is observation, diagnosis or therapy which is to be carried out.

A switching unit 99 produces a command signal 100 or 101 in response to the switching signal 98. In response to the command signal 100, the motor 94 for the optical chopper 93 is energised whereas in response to the command signal 101, shutters 102 and 103 for the optical amplifier 84 and spectroscope 87 are operated.

The mode of operation of the second embodiment is as follows:

Therapy of cancerous focuses 55 using the second embodiment is substantially similar to that described above so that no further description in this connection is necessary.

In order to diagnose whether a focus 55 is cancerous, the wavelength tuning element 14 in the titanium-sapphire laser oscillator 9 shown in Figure 2 is angularly adjusted such that only a laser beam with a wavelength of 820 nm is transmitted and laser beams with other wavelengths are absorbed. (In response to the laser beam with the wavelength of 820 nm, the second harmonic generator 17 produces a laser beam with a wavelength of 410 nm in accordance with relation (1) above.)

HPD is injected into a blood vessel of the patient. If the focus 55 is cancerous, HPD is absorbed by the cancerous tissue. When irradiated by the laser beam with the wavelength of 410 nm, the HPD-laden focus 55 is caused to generate fluorescence with a wavelength of 630 nm.

After the injection of HPD, the fibrescope 80 is positioned such that its tip is substantially in opposing relationship with the focus 55.

The control panel 95 is now manually operated to deliver the command signal 96 and the computer 97 activates the image display unit 82 and issues the switching signal 98 to the switching unit 99. The switching unit 99 outputs the command signal 101 to open the shutters 103 and 102 of the spectroscope 103 and amplifier 84, respectively.

The control panel 95 is also manually operated to deliver the command signal 73 to the YAG laser 52 to activate the solid-state laser unit 51. In response to this signal, the YAG laser oscillator 1 is energised to cause the laser unit 51 to produce a laser beam with a wavelength of 532 nm, as in the first embodiment.

The laser beam with the wavelength of 532 nm enters the laser rod 10 in the titanium-sapphire laser oscillator 9 so that a fundamental wave with a wavelength between 670 and 1100 nm is generated by the rod 10. During resonance of the titanium-sapphire laser fundamental wave between the mirrors 12 and 13, only the titanium-sapphire fundamental wave with a wavelength of 820 nm is transmitted through the wavelength tuning element 14 so that the laser oscillator 9 produces a titanium-sapphire laser fundamental wave with a wavelength of 820 nm.

The fundamental wave thus produced is delivered to the titanium-sapphire second harmonic generator 17 which produces a titanium-sapphire laser second harmonic with a wavelength of 410 nm in accordance with relation (1) above.

The second harmonic thus produced is delivered to the titanium-sapphire third harmonic generator 18 and that proportion of the second harmonic which passes through the generator 18 is separated from the laser beams with other wavelengths by the prism 19.

The laser beam with a wavelength of 410 nm separated by the prism 19 is radiated through the irradiating optical fibre 59 in the fibrescope 80 to the focus 55.

In this case, the HPD-laden focus 55 which is cancerous generates fluorescence with a wavelength of 630 nm.

The spectroscope 87 receives fluorescence from the focus 55 and resolves the fluorescence with a wavelength of 630 nm which is amplified by the optical amplifier 89 and delivered, as the video signal 88, to the image processor 92.

The image processor 92 converts the video signal 88 into the processed video signal 91 which is supplied to the monitor screen 65 so that the spectrum of fluorescence from the focus 55 is graphically displayed.

In order to measure the ratio of the cancerous tissues to the whole focus 55 by the operation of the control panel 95, the shutter 103 of the spectroscope 87 is closed while the shutter 102 of the optical amplifier 84 is opened so that the solid-state laser unit 51 produces a laser beam with a wavelength of 410 nm which is supplied through the fibrescope 80 to irradiate the focus 55.

When the focus 55 is cancerous, it absorbs HPD and generates fluorescence with a wavelength of 630 nm. The optical amplifier 84 receives the fluorescence from the focus 55 and amplifies it to generate the amplified fluorescent signal 83 which is converted to the video signal 85 by the highly-sensitive camera 86. The image processor 92 converts the video signal 85 into the processed video signal 91 which is transmitted to the monitor screen 65, whereby the fluorescent positions on the focus 55 are graphically displayed.

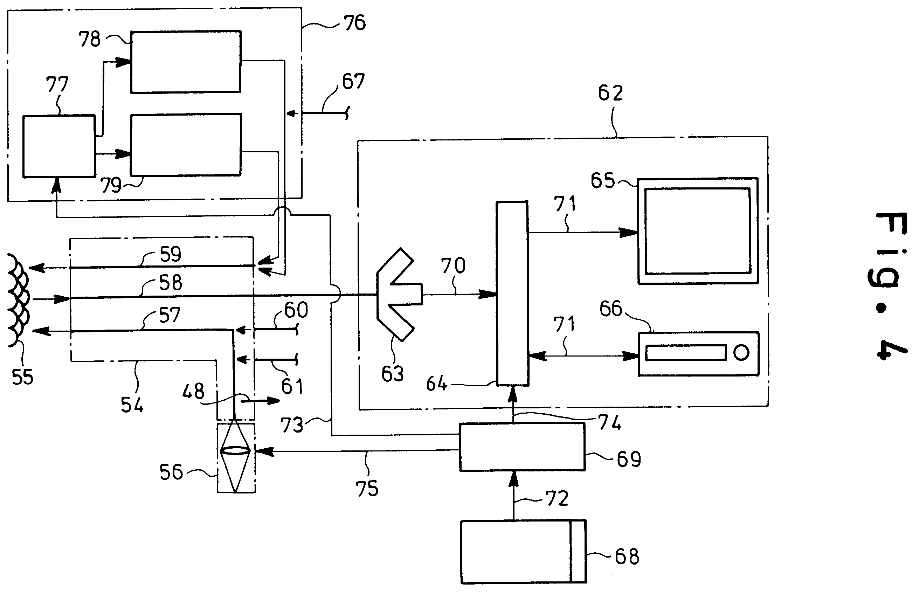

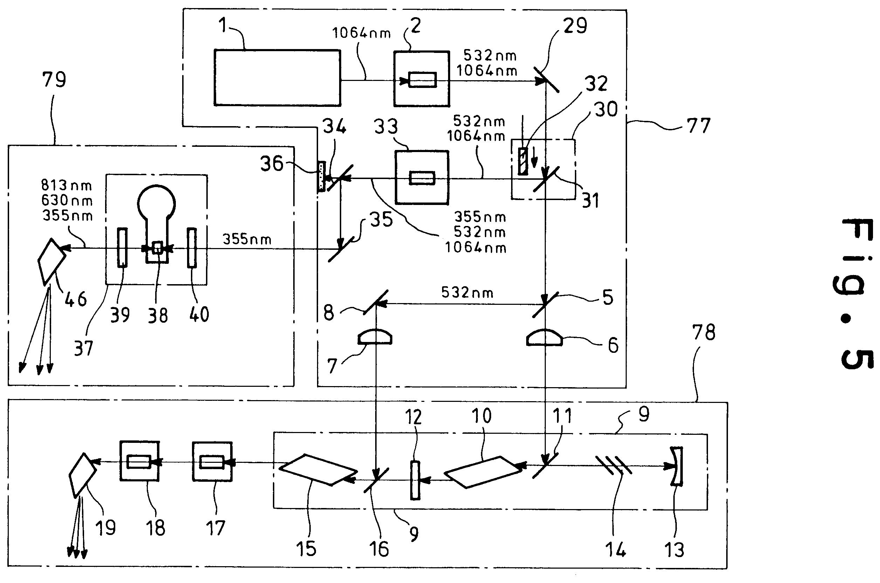

Figures 4 and 5 illustrate a third embodiment of the present invention in accordance with Claim 4. In this embodiment the fibrescope 54, the xenon arc lamp 56, the image display unit 62, the control panel 68 and the controller 69 are substantially similar to those described above with reference to Figures 1 and 2. In Figures 4 and 5, those parts which are similar to those shown in Figures 1 and 2 are designated by the same reference numerals.

A solid-state laser unit generally designated by numeral 76 comprises a YAG laser 77 for producing a laser beam with a predetermined wavelength, a titanium-sapphire laser 78 which is pumped by the laser beam from the YAG laser 77 to produce a laser beam with a predetermined wavelength and an optical parametric oscillator unit 79 which produces a laser beam with a predetermined wavelength from the laser beam generated by the YAG laser 77.

Referring now to Figure 5, the YAG laser 77 in the solid-state laser unit 76 comprises a total reflecting mirror 29 which reflects the YAG laser second harmonic with a wavelength of 532 nm produced by the generator 2 and the YAG laser fundamental wave with a wavelength of 1064 nm which has not been converted into the second harmonic by the generator 20. Situated in the pathway of the reflected beam is an optical-path switch 30 comprising a dichroic polarisation mirror 31 and a beam damper 32 for cutting off the beam reflected from the mirror 31. The YAG laser second harmonic and fundamental wave reflected from the mirror 29 are incident on the mirror 31.

When the reflected beam from the mirror 31 is not cut off by the beam damper 32, the switch 30 reflects the YAG laser second harmonic and fundamental wave incident on the mirror 31. On the other hand, when the reflected beam from the mirror 31 is cut off by the beam damper 32, the optical-path switch 30 reflects only the YAG laser fundamental wave incident on the mirror 31 and transmits the YAG laser second harmonic incident on the mirror 31.

Situated in the pathway of the beam reflected from the mirror 31 is a YAG laser third harmonic generator 33 for producing a laser beam with a wavelength of 355 nm (YAG laser third harmonic) from the YAG laser second harmonic and fundamental wave reflected by the optical-path switch 30 in accordance with relation (2) above. A dichroic mirror 34 passes or transmits only the fundamental wave and the second harmonic, which were not converted by the generator 33, and reflects the third harmonic. A total reflecting mirror 33 reflects the YAG laser third harmonic reflected from the mirror 34 into an optical parametric oscillator with 79 and a beam damper 39 cuts off or attenuates the YAG laser fundamental wave and second harmonic which pass through the mirror 34.

Still referring to Figure 5, the titanium-sapphire laser 78 is substantially similar to that described above with reference to Figure 2 except that the titanium-sapphire laser oscillator 24 for generating a difference frequency, the dichroic mirrors 25 and 26, the difference frequency generator 27 and the pellinbroca prism 28 are eliminated and that the laser 78 incorporates a titanium-sapphire laser oscillator 9, a titanium-sapphire laser second harmonic generator 17, a titanium-sapphire third harmonic generator 18 and a pellinbroca prism 19, all of which are substantially similar to those described above in the first embodiment with reference to Figure 2.

The optical parametric oscillator unit 79 comprises an optical parametric oscillator 37 which incorporates a non-linear optical crystal 38, such as a BBO (Beta-Barium borate) crystal, and front and rear mirrors 39 and 40 and generates a laser beam with a wavelength λ₇ and a laser beam with a wavelength λ₈ from the YAG laser third harmonic reflected from the mirror 35 in accordance with the following relation:$\frac{\text{1}}{{\text{λ}}_{\text{9}}} \text{=} \frac{\text{1}}{{\text{λ}}_{\text{7}}} \text{+} \frac{\text{1}}{{\text{λ}}_{\text{8}}}$ where
- λ₉:: wavelength of the pumping beam, in this case the YAG laser third harmonic,
- λ₇ and λ₈:: wavelengths of the generated beam.
A pellinbroca prism 46 separates the laser beams with the wavelengths λ₇ and λ₈ generated by the oscillator 37 and that proportion of the YAG laser third harmonic which is not converted by the oscillator 37.

In the non-linear optical crystal 38, the pumping wavelength λ₉ in relation (4) automatically determines the wavelengths λ₇ and λ₈ of the generated beams depending upon the angle of the crystal.

The mode of operation is as follows:-

In therapy of a focus 55, the angle of the non-linear optical crystal 38 shown in Figure 5 is so determined that one of λ₇ and λ₈ in relation (4) is 630 nm and the beam damper 32 of the optical-path switch 30 is set at the inoperative position such that reflection of the laser beam by the mirror 31 is not interrupted.

Under these conditions, when the YAG laser fundamental wave with a wavelength of 1064 nm is generated by the YAG laser oscillator 1, the YAG laser second harmonic generator 2 generates the YAG laser second harmonic with a wavelength of 532 nm from the YAG laser fundamental wave. The YAG laser second harmonic thus generated and the YAG laser fundamental wave which pass through the YAG laser second harmonic generator 2 are redirected to the YAG laser third harmonic generator 33 by the mirrors 29 and 31 so that the generator 33 generates the YAG laser third harmonic with a wavelength of 355 nm in accordance with relation (2) above from the YAG laser fundamental wave and the second harmonic.

That proportion of the fundamental wave and second harmonic which pass through the generator 33 pass through the mirror 34 and are attenuated by the beam damper 36 while the third harmonic produced by the generator 33 is reflected by the mirrors 34 and 35 to the optical parametric oscillator 37. In response to the incident third harmonic the oscillator 37 generates laser beams with wavelengths of 630 nm and 813 nm, respectively, and a proportion of the third harmonic passes through the oscillator 37. The laser beams with the wavelengths of 630 nm and 813 nm, respectively, generated by the optical parametric oscillator 37 and the YAG laser third harmonic which passes through the oscillator 37 are directed to and separated from each other by the prism 46. The laser beam with the wavelength of 630 nm separated by the prism 46 is administered through the irradiating optical fibre 59 in the fibrescope 54 to irradiate the focus 55 so that the HPD-laden cancerous tissue is destroyed by it.

The third embodiment has been described with reference to the use of HPD. If another photosensitive substance is used, the angle of the non-linear crystal 38 is varied to match the wavelength of one of the laser beams generated by the optical parametric oscillator 37 with the characteristics or absorption band of the photosensitive substance used.

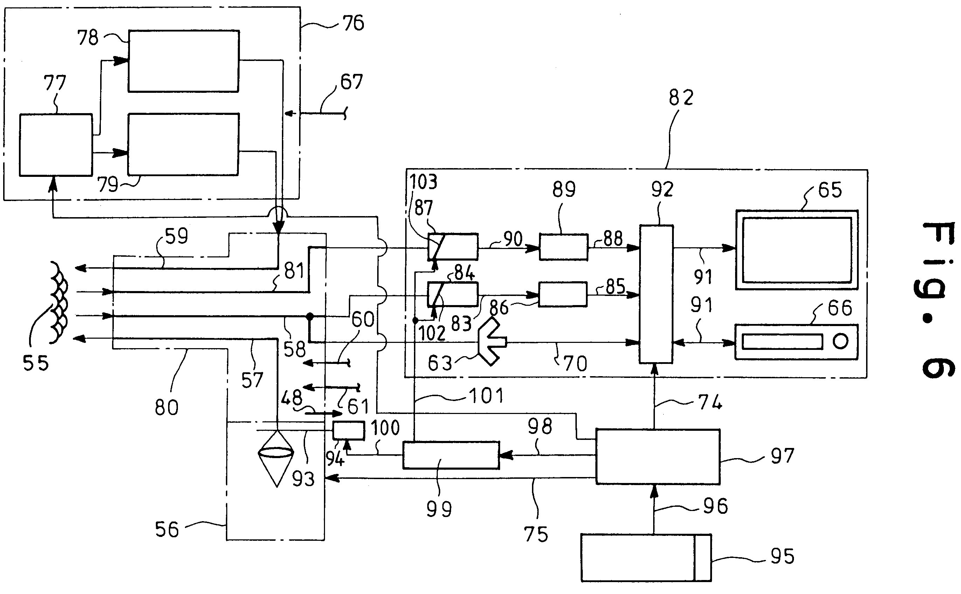

Figure 6 illustrates a fourth embodiment of the present invention which is claimed in Claims 5 and 6. The fourth embodiment is substantially similar to the second embodiment shown in Figure 3 except that the solid-state laser unit 51 is replaced by the solid-state laser unit 76 shown in Figure 4. The fourth embodiment also has novel features substantially similar to those of the second embodiment described above with reference to Figure 3.

## Claims

1. A cancer diagnosis and therapy system for a cancerous focus that has absorbed a photosensitive substance comprising:
a solid state laser unit (76) including a YAG laser oscillator (1) for producing a YAG laser fundamental wave;
a YAG laser second harmonic generator (2) for producing a YAG laser second harmonic from the fundamental wave produced by the oscillator (1);
a YAG laser third harmonic generator (33) for generating the YAG laser third harmonic;
an optical parametric oscillator (37) for generating an optical parametric oscillator laser beam from the YAG laser third harmonic generated by the generator (33);
the optical parametric oscillator laser beam being of such a wavelength to destroy the photosensitive substance laden cancerous focus; and
a fibrescope (54; 80) for irradiating cancerous focuses (55) with the laser beam generated by the laser unit (76) and observing the focuses (55) and an image display unit (62; 82) for viewing the focuses (55) through the fibrescope (54) and displaying an image of the focuses (55) characterised in that:
the YAG laser third harmonic generator (33) generates the YAG laser third harmonic from that proportion of the YAG laser fundamental wave which is not converted to the second harmonic by the generator (2) and from the YAG second harmonic generated by the generator (2), and the solid state laser unit (76) further comprises a titanium-sapphire laser oscillator (9) which is pumped by the YAG laser second harmonic generator (2) to generate a titanium-sapphire laser fundamental wave, a titanium-sapphire laser second harmonic generator (17) for generating a titanium-sapphire laser second harmonic from the titanium-sapphire laser fundamental wave generated by the oscillator (9), the titanium-sapphire laser second harmonic being of such a wavelength to cause fluorescence of the photosensitive substance.

2. A system as claimed in claim 1 characterised in that the image display unit (82) is arranged to graphically display the spectrum of fluorescence from the focuses (55).

3. A system as claimed in claim 1 or claim 2 characterised in that the image display unit (82) is arranged to graphically display fluorescent positions on the focuses (55).

## Patentansprüche

1. Krebsdiagnose- und -therapiesystem für einen Krebsherd, der eine lichtempfindliche Substanz absorbiert hat, mit:
einer Festkörperlaser-Einheit (76), die einen YAG-Laseroszillator (1) enthält, um eine YAG-Laser-Grundwelle zu erzeugen;
einem Generator (2) für die zweite Harmonische des YAG-Lasers, der eine zweite Harmonische des YAG-Lasers aus der vom Oszillator (1) erzeugten Grundwelle erzeugt;
einem Generator (33) für die dritte Harmonische des YAG-Lasers, der die dritte Harmonische des YAG-Lasers erzeugt;
einem optischen parametrischen Oszillator (37), der aus der vom Generator (33) erzeugten dritten Harmonischen des YAG-Lasers einen Laserstrahl eines optischen parametrischen Oszillators erzeugt;
wobei der Laserstrahl des optischen parametrischen Oszillators eine Wellenlänge besitzt, derart, daß die im Krebsherd vorhandene lichtempfindliche Substanz zerstört wird; und
einem Faserskop (54; 80), um Krebsherde (55) mit dem Laserstrahl zu bestrahlen, der von der Lasereinheit (76) erzeugt wird, und um die Herde (55) zu beobachten, und einer Bildanzeigeeinheit (62; 82), um die Herde (55) durch das Faserskop (54) zu beobachten und ein Bild der Herde (55) anzuzeigen, dadurch gekennzeichnet, daß:
der Generator (33) für die dritte Harmonische des YAG-Lasers die dritte Harmonische des YAG-Lasers aus demjenigen Anteil der Grundwelle des YAG-Lasers, der nicht vom Generator (2) in die zweite Harmonische umgesetzt wird, und aus der vom Generator (2) erzeugten zweiten Harmonischen des YAG-Lasers erzeugt, und daß die Festkörperlaser-Einheit (76) ferner einen Titan-Saphir-Laseroszillator (9), der mittels des Generators (2) für die zweite Harmonische des YAG-Lasers gepumpt wird, um eine Grundwelle des Titan-Saphir-Lasers zu erzeugen, sowie einen Generator (17) für die zweite Harmonische des Titan-Saphir-Lasers enthält, der aus der vom Oszillator (9) erzeugten Grundwelle des Titan-Saphir-Lasers eine zweite Harmonische des Titan-Saphir-Lasers erzeugt, wobei die zweite Harmonische des Titan-Saphir-Lasers eine Wellenlänge besitzt, die eine Fluoreszenz der lichtempfindlichen Substanz hervorruft.

2. System nach Anspruch 1, dadurch gekennzeichnet, daß die Bildanzeigeeinheit (82) so beschaffen ist, daß sie das Fluoreszenzspektrum von den Herden (55) graphisch anzeigt.

3. System nach Anspruch 1 oder Anspruch 2, dadurch gekennzeichnet, daß die Bildanzeigeeinheit (82) so beschaffen ist, daß sie Fluoreszenzpositionen auf den Herden (55) graphisch anzeigt.

## Revendications

1. Système de diagnostic et de thérapie du cancer destiné à un foyer cancéreux ayant absorbé une substance photosensible comprenant :
une unité à laser monolithique (76) comprenant un oscillateur de laser YAG (yttrium-aluminium-grenat) (1) destiné à produire une onde fondamentale de laser YAG,
un générateur de seconde harmonique de laser YAG (2) destiné à produire une seconde harmonique de laser YAG à partir de l'onde fondamentale produite par l'oscillateur (1),
un générateur de troisième harmonique de laser YAG (33) destiné à générer la troisième harmonique du laser YAG,
un oscillateur paramétrique optique (37) destiné à générer un faisceau laser d'oscillateur paramétrique optique à partir de la troisième harmonique de laser YAG, générée par le générateur (33),
le faisceau laser d'oscillateur paramétrique optique étant d'une longueur d'onde telle qu'elle détruit le foyer cancéreux chargé de substance photosensible, et
un fibroscope (54, 80) destiné à illuminer les foyers cancéreux (55) avec le faisceau laser généré par l'unité à laser (76) et à observer les foyers (55), et une unité d'affichage d'image (62, 82) destinée à observer les foyers (55) par l'intermédiaire du fibroscope (54) et à afficher une image des foyers (55), caractérisé en ce que :
le générateur de troisième harmonique de laser YAG (33) génère la troisième harmonique du laser YAG à partir de la proportion de l'onde fondamentale du laser YAG qui n'est pas convertie en seconde harmonique par le générateur (2) et à partir de la seconde harmonique de laser YAG générée par le générateur (2), et l'unité à laser monolithique (76) comprend en outre un oscillateur de laser titane-saphir (9) qui est pompé par le générateur de seconde harmonique de laser YAG (2) afin de générer une onde fondamentale de laser titane-saphir, un générateur de second harmonique de laser titane-saphir (17) destiné à générer une seconde harmonique de laser titane-saphir à partir de l'onde fondamentale du laser titane-saphir générée par l'oscillateur (9), la seconde harmonique du laser titane-saphir étant d'une longueur d'onde telle qu'elle provoque la fluorescence de la substance photosensible.

2. Système selon la revendication 1, caractérisé en ce que l'unité d'affichage d'image (82) est agencée pour afficher graphiquement le spectre de fluorescence provenant des foyers (55).

3. Système selon la revendication 1 ou la revendication 2, caractérisé en ce que l'unité d'affichage d'image (82) est agencée pour afficher graphiquement des positions fluorescentes sur les foyers (55).
